# EUROPEAN PATENT SPECIFICATION

(11) **EP 1 293 997 B1**
(45) Date of publication and mention of the grant of the patent: **28.05.2008**
(21) Application number: 02019739.8
(22) Date of filing: 03.09.2002
(51) Int. Cl.: H01G 9/20, H01M 6/16, C07F 7/08

(54) **Imidazolium compounds and electrolytic composition for a dye sensitized solar cell**
Imidazolverbindungen und Elektrolytzusammensetzung für eine Farbstoffsolarzelle
Composés d' imidazolium et composition d'électrolyte pour une cellule solaire sensibilisée par des colorants

(30) Priority: 18.09.2001 JP 2001283172
(43) Date of publication of application: 19.03.2003
(73) Proprietor: FUJIFILM Corporation, Minato-ku Tokyo (JP)
(72) Inventor: Watanabe, Tetsuya, Minami-Ashigara-shi, Kanagawa-ken (JP)
(74) Representative: Patentanwälte Dr. Solf & Zapf

(56) References cited:
- WO-A-95/18456
- WO-A-99/28292
- US-B1- 6 221 941

## Description

### FIELD OF THE INVENTION

The present invention relates to a dye-sensitized solar cell based on a photoelectric conversion device comprising an electrolytic composition for exhibiting excellent photoelectric conversion properties.

### BACKGROUND OF THE INVENTION

Liquid electrolyte compositions (electrolyte solutions) comprising solvents and electrolyte salts dissolved therein have conventionally been used as electrolyte compositions for electrochemical devices such as cells, capacitors, sensors, display devices, recording devices, etc. However, the electrochemical devices using such liquid electrolyte compositions are unreliable because they often suffer from the leakage of liquid electrolyte compositions during long-term operation or storage.

"Nature," Vol. 353, pages 737 to 740 (1991), U.S. Patent 4,927,721, etc. disclose photoelectric conversion devices utilizing fine semiconductor particles sensitized by dyes, and photoelectric cells comprising them. Because the photoelectric conversion devices also comprise the electrolyte solutions in charge transfer layers, however, there is still likelihood that their photoelectric conversion efficiency is extremely lowered, or their photoelectric conversion function is lost by leakage or depletion of the electrolyte solutions during the long-term operation or storage.

Proposed under these circumstances, WO 93/20565 discloses a photoelectric conversion device comprising a solid electrolyte. Further, Nippon Kagaku Kaishi (the Japanese Journal of Chemistry), 7, 484 (1997), JP 7-288142 A, "Solid State Ionics," 89, 263 (1986), and JP 9-27352 A are photoelectric conversion devices using solid electrolytes comprising cross-linked polyethylene oxide high-molecular compounds. However, the devices using the solid electrolytes are insufficient in photoelectric conversion characteristics, especially in short-circuiting current density.

Disclosed in WO 95/18456, JP 8-259543 A, "Denki Kagaku (Electrochemistry)," 65, 11, 923 (1997), etc. are photoelectric conversion devices comprising electrolyte compositions in which pyridinium salts, imidazolium salts, triazolium salts, etc. are used as the electrolytes to prevent the leakage and depletion of the electrolyte compositions. Because the salts are in a liquid state at room temperature (around 25°C), they are called "room-temperature-molten salt." In such electrolyte compositions, solvents for dissolving the electrolytes such as water and organic solvents are needed in little amounts, if necessary, whereby the photoelectric conversion devices comprising the compositions exhibit improved durability. However, the devices using conventional, room-temperature-molten salts are disadvantageously insufficient in photoelectric conversion efficiency.

US 6.221.941 describes weakly coordinating polyfluoroalkoxide anions.

### OBJECTS OF THE INVENTION

An object of the present invention is to provide an electrolytic composition excellent in charge-transporting capability suitable for a dye-sensitized solar cell.

Another object of the present invention is to provide a photoelectric conversion device comprising such an electrolytic composition for exhibiting excellent photoelectric conversion properties.

A further object of the present invention is to provide a dye-sensitized solar cell comprising such a photoelectric conversion device.

### SUMMARY OF THE INVENTION

As a result of intense research in view of the above objects, the inventor has found that an electrolytic composition comprising an electrolyte salt composed of a cation and an anion having a silicon atom is excellent in charge-transporting capability. The present invention has been accomplished by this finding.

Thus, the electrolytic composition suitable for a dye-sensitized solar cell of the present invention comprises an electrolyte salt represented by the following general formula (I):

(Q₁)ₙ₁·(X)ₙ₂ (I),

wherein Q₁ represents an unsubstituted imidazolium cation, X represents an anion having a silicon atom, and each of n₁ and n₂ represents an integer of 3. The electrolytic composition of the present invention is preferably used for a photoelectric cell or a solar cell.

X is preferably represented by the following general formula (II): 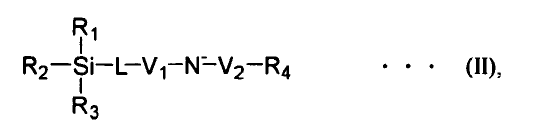 wherein each of R₁ to R₄ independently represents a substituent of a substituted or unsubstituted alkyl group having 1 to 2 carbon atoms, each of L, V₁ and V₂ independently represents a bivalent linking group, and at least one of V₁ and V₂ represents -CO-, -SO-, -SO₂- or -PO(OR₅)-, wherein R₅ represents methyl group. At least one of V₁ and V₂ is more preferably -SO₂-

Q₁ is preferably an organic cation having positive charge on a nitrogen atom.

The solvent content of the electrolytic composition of the present invention is preferably 10% by mass or less based on the entire electrolytic composition. The electrolytic composition preferably further comprises an iodine salt and/or iodine.

The photoelectric conversion device of the present invention comprises a conductive support, a photosensitive layer (20), a charge transfer layer (30) and a counter electrode (40), the charge transfer layer (30) comprising the electrolytic composition of the present invention. The photosensitive layer (20) preferably comprises fine semiconductor particles (21) sensitized by a dye (22). The dye-sensitized solar cell of the present invention comprises such a photoelectric conversion device.

In the present invention, a novel compound represented by the following general formula (III) is preferably used as an electrolyte salt.

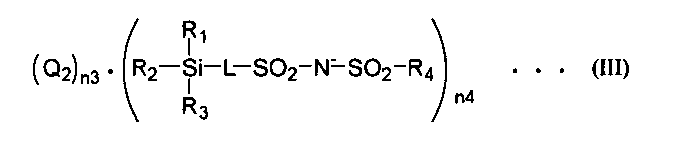

In the general formula (III), Q₂ represents an unsubstituted imidazolium cation, each of R₁ to R₄ independently represents a substituent of a substituted or unsubstituted alkyl group having to 2 carbon atoms, L represents a bivalent linking group, and each of n₃ and n₄ represents an integer of 1.

### BRIEF DESCRIPTION OF THE DRAWINGS

Fig. 1 is a partial cross sectional view showing a preferable structure of the photoelectric conversion device of the present invention;
Fig. 2 is a partial cross sectional view showing another preferable structure of the photoelectric conversion device of the present invention;
Fig. 3 is a partial cross sectional view showing a further preferable structure of the photoelectric conversion device of the present invention;
Fig. 4 is a partial cross sectional view showing a still further preferable structure of the photoelectric conversion device of the present invention;
Fig. 5 is a partial cross sectional view showing a still further preferable structure of the photoelectric conversion device of the present invention;
Fig. 6 is a partial cross sectional view showing a still further preferable structure of the photoelectric conversion device of the present invention;
Fig. 7 is a partial cross sectional view showing a still further preferable structure of the photoelectric conversion device of the present invention;
Fig. 8 is a partial cross sectional view showing a still further preferable structure of the photoelectric conversion device of the present invention; and
Fig. 9 is a partial cross sectional view showing a still further preferable structure of the photoelectric conversion device of the present invention.

### DETAILED DESCRIPTION OF THE PREFERRED EMBODIMENTS

### [1] Electrolytic composition

The electrolytic composition of the present invention comprises an electrolyte salt represented by the following general formula (I). Further, the electrolytic composition may comprise an iodine salt and/or iodine and a solvent, etc. This electrolytic composition is usable for a reaction solvent for chemical reactions, metal plating, etc., CCD (charge-coupled device) cameras, various photoelectric conversion devices, cells, etc. The composition is preferably used for lithium ion secondary batteries or photoelectric cells, particularly those comprising semiconductors. The constituent elements of the electrolytic composition of the present invention will be described in detail below.

### (A) Electrolyte salt

The electrolytic composition of the present invention comprises an electrolyte salt represented by the following general formula (I):

(Q₁)ₙ₁·(X)ₙ₂ (I).

The electrolyte salt represented by the general formula (I) is referred to as "electrolyte salt (I)" hereinafter.

The electrolyte salt (I) is usually a low-melting point salt, which is called "molten salt." The melting point of the electrolyte salt (I) is preferably 100°C or lower, more preferably 80°C or lower, particularly 60°C or lower. Such electrolyte salt may be a salt in a liquid state around at room temperature, which is called "room temperature-molten salt."

The electrolyte salt (I) is likely to be used as an electrolytic composition with a little amount of a solvent, though a solvent is not needed in some cases. When the electrolyte salt (I) is solid at room temperature (around 25°C), a solvent and an additive, etc. can be added thereto to use as a liquid electrolyte composition. It may also be incorporated into a photoelectric conversion device without additives, by a method for melting it by heat to cause it to penetrate into an electrode, a method for causing it to penetrate into an electrode using a low-boiling point solvent such as methanol, acetonitrile, dichloromethane, etc., and then removing the solvent by heating, etc.

In the general formula (I), Q₁ represents an unsubstituted imidazolium cation. Q₁ is an organic cation. When Q₁ is an organic cation, Q₁ is preferably a cation having positive charge on a nitrogen atom such as an imidazolium cation.

In the general formula (I), each of n₁ and n₂ represents an integer of 1.

In the general formula (I), X is not limited as long as it is an anion comprising a silicon atom, though X is preferably represented by the following general formula (II):

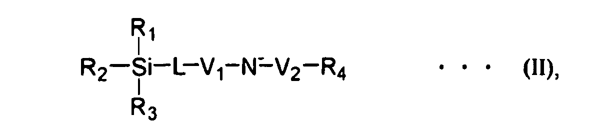

In the general formula (II), each of R₁ to R₄ independently represents a substituent. This substituent is preferably a substituted or unsubstituted alkyl group preferably having 1 to 2 carbon atoms, such as a methyl group, an ethyl group.

In the general formula (II), L represents a bivalent linking group. L is preferably composed of atoms selected from the group consisting of carbon, hydrogen oxygen. L is a group having preferably 1 to 4 carbon atoms.

In the general formula (II), each of V₁ and V₂ represents a bivalent linking group, at least one of V₁ and V₂ represents -CO-, -SO-, -SO₂- or -PO(OR₅)-. R₅ represents methyl group. At least one of V₁ and V₂ is preferably -SO₂-. More preferably both of V₁ and V₂ are -SO₂-.

In the present invention, a novel compound represented by the following general formula (III) can be preferably used as the electrolyte salt (I).

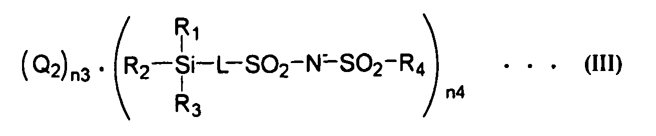

In the general formula (III), Q₂ represents an unsubstituted imidazolium cation.

In the general formula (III), R₁ to R₄ and L are the same as those in the above general formula (II) in their definitions.

In the general formula (III), each of n₃ and n₄ represents an integer of 1.

The content of the electrolyte salt (I) in the electrolytic composition of the present invention is preferably 10% by mass or more, more preferably 20 to 95% by mass, based on the entire electrolytic composition. Specific examples of the electrolyte salt (I) will be shown without limitation.

| | |
|---|---|
| 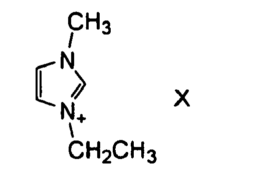 | |

| | X |
|---|---|
| D-1 | (CH₃)₃SiCH₂CH₂CH₂SO₂N⁻SO₂CF₃ |
| D-2 | (CH₃)₃SiCH₂CH₂SO₂N⁻SO₂CF₃ |
| D-3 | (CH₃)₃SiCH₂CH₂CON⁻SO₂CF₃ |
| D-4 | (CH₃)₃SiCH₂CH₂N⁻SO₂CF₃ |
| D-5 | (CH₃)₃SiCH₂CH₂CH₂SON⁻SO₂CF₃ |
| D-6 | 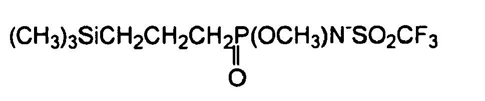 |
| D-7 | (C₂H₅)₃SiCH₂CH₂SO₂N⁻SO₂CF₃ |
| D-8 | (CH₃)₃SiCH₂CH₂OCH₂CH₂SO₂N⁻SO₂CF₃ |
| D-9 | 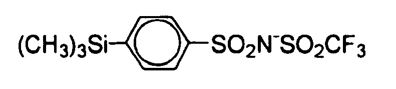 |
| D-10 | 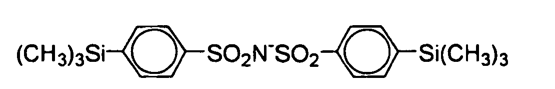 |

Also, reference examples of the electrolyte salt (I) comprising an unsubstituted pyridinium cation as Q₁ will be shown below.

| | |
|---|---|
| 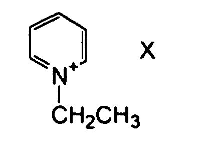 | |

| | X |
|---|---|
| D-11 | (CH₃)₃SiCH₂CH₂CH₂SO₂N⁻SO₂CF₃ |
| D-12 | (CH₃)₃SiCH₂CH₂CON⁻SO₂CF₃ |
| D-13 | (CH₃)₃SiCH₂CH₂N⁻SO₂CF₃ |
| D-14 | 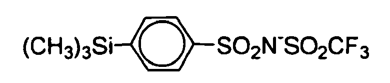 |
| D-15 | (n-C₄H₉)₄N⁺ · (CH₃)₃SiCH₂CH₂CH₂SO₂N⁻SO₂CF₃ |
| D-16 | (n-C₄H₉)₄N⁺ · (CH₃)₃SiCH₂CH₂CON⁻SO₂CF₃ |
| D-17 | 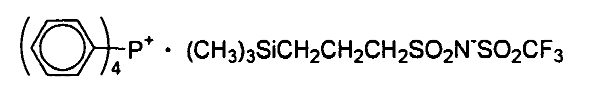 |
| D-18 | Li⁺ · (CH₃)₃SiCH₂CH₂CH₂SO₂N⁻SO₂CF₃ |
| D-19 | Na⁺ · (CH₃)₃SiCH₂CH₂CON⁻SO₂CF₃ |
| D-20 | Mg²⁺ · {(CH₃)₃SiCH₂CH₂SO₂N⁻SO₂CF₃}₂ |
| D-21 | 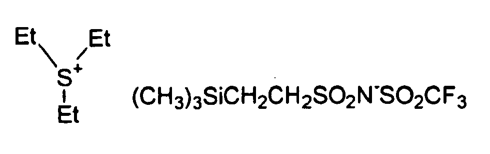 |

The electrolyte salt (I) may be synthesized by a common method, and may be synthesized easily by a method, for instance, described in Inorganic Chemistry, 35 (1996) pages 1168-1178.

### (B) Iodine salt and iodine

When the electrolytic composition of the present invention is used for a photoelectric conversion device, the electrolytic composition preferably comprises an iodine salt and/or iodine. Such iodine salts include pyridinium salts, imidazolium salts, triazolium salts, etc. described in WO 95/18456, JP 8-259543 A, "Denki Kagaku (Electrochemistry)," 65, 11, 923 (1997), etc. The electrolytic composition of the present invention may further comprise another salt in addition to the above electrolyte salt (I) and an iodine salt.

The iodine salt content in the electrolytic composition of the present invention is preferably 10% by mass or more, more preferably 50 to 95% by mass based on the entire electrolytic composition. When iodine is added to the electrolytic composition, the iodine content is preferably 0.1 to 20% by mass, more preferably 0.5 to 5% by mass based on the entire electrolytic composition.

### (C) Solvent

The electrolytic composition of the present invention may comprise a solvent. The solvent content in the electrolytic composition is preferably 50% by mass or less, more preferably 30% by mass or less, particularly 10% by mass or less, based on the entire electrolyte composition.

Preferably used in the present invention is a solvent having excellent ion conductibility because of low viscosity and high ion mobility, and/or high dielectric constant and effective carrier concentration. Examples of such solvents include carbonates such as ethylene carbonate and propylene carbonate; heterocyclic compounds such as 3-methyl-2-oxazolidinone; ethers such as dioxan and diethyl ether; linear ethers such as ethylene glycol dialkylethers, propylene glycol dialkylethers, polyethylene glycol dialkylethers and polypropylene glycol dialkylethers; alcohols such as methanol, ethanol, ethylene glycol monoalkylethers, propylene glycol monoalkylethers, polyethylene glycol monoalkylethers and polypropylene glycol monoalkylethers; glycols such as ethylene glycol, propylene glycol, polyethylene glycol, polypropylene glycol and glycerin; nitriles such as acetonitrile, glutarodinitrile, methoxyacetonitrile, propionitrile, benzonitrile and biscyanoethyl ether; esters such as carboxylates, phosphates and phosphonates; aprotic polar solvents such as dimethylsulfoxide (DMSO) and sulfolane; water; etc. Two or more of these solvents may be mixed together.

### (D) Others

The electrolytic composition of the present invention may be used in the form of a gel or a solid, which is obtained by adding a polymer or an oil-gelling agent, by polymerization together with multifunctional monomers or by a cross-linking reaction, etc.

In the case of gelling the electrolytic composition by adding a polymer, polymers described in "Polymer Electrolyte Reviews-1 and 2", edited by J. R. MacCallum and C. A. Vincent, ELSEVIER APPLIED SCIENCE may be used. Polyacrylonitrile or polyvinylidene fluoride is preferably used.

In the case of gelling the electrolytic composition by adding an oil-gelling agent, the oil-gelling agents described in J. Chem. Soc. Japan, Ind. Chem. Soc., 46, 779 (1943), J. Am. Chem. Soc., 111, 5542 (1989), J. Chem. Soc., Chem. Commun., 390 (1993), Angew. Chem. Int. Ed. Engl., 35, 1949 (1996), Chem. Lett., 885 (1996), J. Chem. Soc., Chem. Commun., 545 (1997), etc. may be used. Preferable among them are those having an amide structure.

When a gel electrolytic composition is formed by the polymerization of a multifunctional monomer, it is preferable to prepare a solution comprising a multifunctional monomer, a polymerization initiator, the electrolytic composition and a solvent, and form a sol electrolyte layer on a photosensitive layer by casting, coating, soaking, impregnation, etc., and then radical-polymerize the multifunctional monomer. The multifunctional monomer preferably has two or more ethylenically unsaturated groups. Preferable examples thereof include divinyl benzene, ethyleneglycol diacrylate, ethyleneglycol dimethacrylate, diethylene glycol diacrylate, diethylene glycol dimethacrylate, trimethylene glycol diacrylate, trimethylene glycol dimethacrylate, pentaerythritol triacrylate and trimethylolpropane triacrylate.

When a mixture of a multifunctional monomer and a monofunctional monomer is used, the amount of the multifunctional monomer in all the monomers is preferably 0.5 to 70% by mass, more preferably 1.0 to 50% by mass.

The above-described monomers can be polymerized by a usual radical polymerization method described in Takayuki Otsu and Masaetsu Kinoshita, "Experiment Method of Polymer Synthesis" issued by Kagaku Dojin, Takayuki Otsu, "Theory of Polymerization Reaction 1, Radical Polymerization (I)" issued by Kagaku Dojin, etc. The radical polymerization may be carried out by a heating method, a light- or electron beam- irradiation method, or an electrochemical method. Preferable among them is heat polymerization. The radical polymerization may be carried out with a polymerization initiator.

The weight ratio of the monomers accounting for the gel electrolytic composition is preferably in the range of 0.5 to 70% by mass, more preferably 1.0 to 50% by mass.

When the gelation of the electrolytic composition is accomplished by the cross-linking reaction of a polymer, a polymer containing a group having cross-linking reactivity is preferably added to the composition together with a cross-linking agent. Groups having cross-linking reactivity are preferably nitrogen-containing heterocyclic groups such as a pyridyl group, an imidazolyl group, a thiazolyl group, an oxazolyl group, a triazolyl group, a morpholyl group, a piperidyl group, a piperazyl group, etc. A preferred cross-linking agent is an electrophilic agent having a plurality of functional groups that can be attacked by a nitrogen atom, for example, multi-functional alkyl halides, aralkyl halides, sulfonates, acid anhydrides, acyl chlorides and isocyanates.

The electrolytic composition of the present invention may comprise a metal iodide such as LiI, NaI, KI, CsI and CaI₂; a metal bromide such as LiBr, NaBr, KBr, CsBr and CaBr₂; a quaternary ammonium bromide such as a tetralkylammonium bromide and pyridinium bromide; a metal complex such as a ferrocyanide-ferricyanide and a ferrocene-ferricinium ion; a sulfur compound such as sodium polysulfide and alkylthiol-alkyldisulfide; a viologen dye; hydroquinone-quinone; etc. These compounds may be used in combination.

The electrolytic composition of the present invention may further comprise a basic compound such as *t*-butylpyridine, 2-picoline, 2,6-lutidine, etc. described in J. Am. Ceram. Soc., 80, (12), 3157-3171 (1997). The concentration of the basic compound is preferably 0.05 to 2 M based on the electrolyte composition.

### [2] Photoelectric conversion device

The photoelectric conversion device of the present invention has an electrically conductive layer, a photosensitive layer, a charge transfer layer and a counter electrode. The charge transfer layer comprises the above-mentioned electrolytic composition of the present invention.

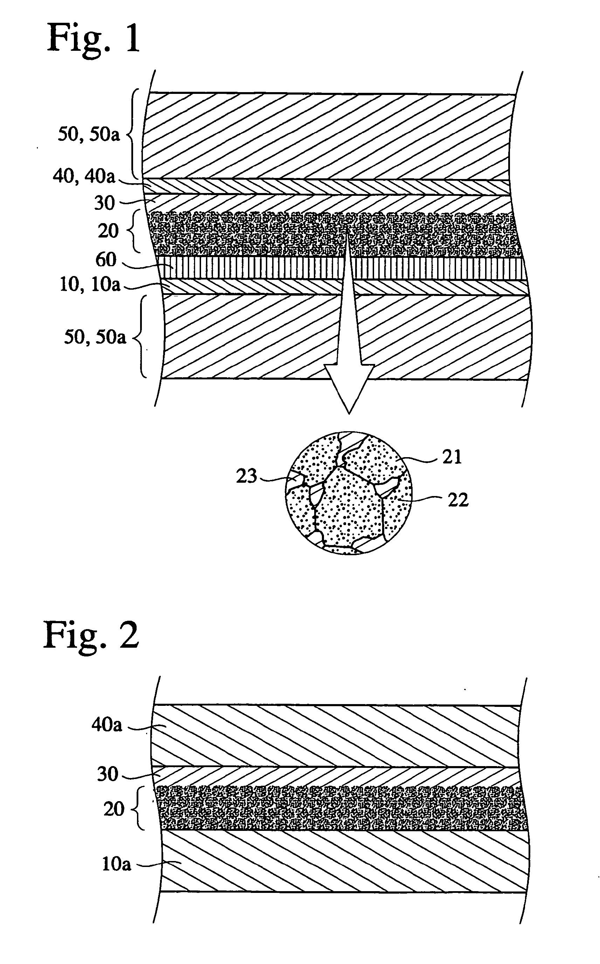

As shown in Fig. 1, the photoelectric conversion device of the present invention preferably has a lamination structure comprising an electrically conductive layer 10, an undercoating layer 60, a photosensitive layer 20 comprising fine semiconductor particles 21 sensitized by a dye 22 and an charge-transporting material 23 penetrating into voids among the fine semiconductor particles 21, a charge transfer layer 30, and a counter electrode layer 40 in this order. The charge-transporting material 23 is preferably the same as the electrolytic composition used in the charge transfer layer 30. The electrically conductive layer 10 and/or the counter electrode layer 40 may be supported by a substrate 50 to improve the strength of the photoelectric conversion device. A layer composed of the electrically conductive layer 10 and the substrate 50 optionally used for supporting it is referred to as "conductive support," and a layer composed of the counter electrode layer 40 and the substrate 50 optionally used for supporting it is referred to as "counter electrode" hereinafter. The electrically conductive layer 10, the counter electrode layer 40 and the substrate 50 shown in Fig. 1 may be transparent.

In the case of using fine *n*-type semiconductor particles in the photoelectric conversion device of the present invention shown in Fig. 1, a light injected to the photosensitive layer 20 excites the dye 22, etc. to generate excited high-energy electrons, which are transported to a conduction band of the fine semiconductor particles 21, and are diffused to reach the electrically conductive layer 10. At this time, the dye 22 is in an oxidized form. In a photoelectric cell, electrons in the electrically conductive layer 10 return to the oxidized dye through the counter electrode layer 40 and the charge transfer layer 30 while doing job in an outside circuit, so that the dye 22 is regenerated. The photosensitive layer 20 generally acts as a negative electrode (photo-anode). The counter electrode layer 40 acts as a positive electrode. In a boundary of adjacent layers such as the electrically conductive layer 10 and the photosensitive layer 20; the photosensitive layer 20 and the charge transfer layer 30; the charge transfer layer 30 and the counter electrode layer 40; etc., components of each layer may be diffused and mixed. Each of the layers will be explained in detail below.

### (A) Conductive support

The conductive support is: (1) a single layer of the electrically conductive layer, or (2) two layers of the electrically conductive layer and the substrate. In the case (1), the electrically conductive layer is preferably made of a material having such a strength that it can fully seal the photoelectric conversion device, for example, a metal such as platinum, gold, silver, copper, zinc, titanium, aluminum and an alloy thereof. In the case (2), a substrate having the electrically conductive layer containing an electrically conductive material formed on the photosensitive layer side may be used. Preferable examples of the electrically conductive materials include metals such as platinum, gold, silver, copper, zinc, titanium, aluminum, indium and alloys thereof; carbon; electrically conductive metal oxides such as indium-tin composite oxides and tin oxides doped with fluorine or antimony; etc. The electrically conductive layer preferably has a thickness of 0.02 to 10 µm.

The surface resistance of the conductive support is desirably as low as possible. The surface resistance of the conductive support is preferably 50 Ω/square or less, more preferably 20 Ω/square or less.

When light is irradiated from the conductive support side, it is preferable that the conductive support is substantially transparent. The term "substantially transparent" used herein means that 10 % or more of light transmittance is obtained in part or all of a range from a visible light to a near infrared light (400-1200 nm). The light transmittance is preferably 50 % or more, more preferably 80 % or more. In particular, light transmittance is preferably high in a wavelength range in which the photosensitive layer has sensitivity.

The transparent conductive support is preferably provided by forming the transparent electrically conductive layer comprising an electrically conductive metal oxide on the transparent substrate of such material as a glass and a plastic by coating or vapor deposition. The transparent electrically conductive layer is preferably made of tin dioxide doped with fluorine or antimony, or indium-tin oxide (ITO). The transparent substrate may be made of glass such as low-cost soda glass excellent in strength and a non-alkali glass suffering from no alkaline elution. In addition, a transparent polymer film may also be used as the transparent substrate. Materials usable for the transparent polymer film are triacetylcellulose (TAC), polyethylene terephthalate (PET), polyethylene naphthalate (PEN), syndiotactic polystyrene (SPS), polyphenylenesulfide (PPS), polycarbonate (PC), polyarylate (PAr), polysulfone (PSF), polyestersulfone (PES), polyimide (PI), polyetherimide (PEI), cyclic polyolefin, a brominated phenoxy resin, etc. To secure sufficient transparency, the amount of the electrically conductive metal oxide coated is preferably 0.01 to 100 g per 1 m² of the glass or plastic substrate.

It is preferable to use a metal lead to reduce the resistance of the transparent conductive support. The metal lead is preferably made of a metal such as platinum, gold, nickel, titanium, aluminum, copper, silver, etc. The metal lead is preferably formed on the transparent substrate by a vapor deposition method, a sputtering method, etc., preferably with a transparent electrically conductive layer comprising conductive tin oxide or ITO formed thereon. Decrease in incident light quantity by the metal lead is suppressed to preferably 10 % or less, more preferably 1 to 5 %.

### (B) Photosensitive layer

The photosensitive layer comprises fine semiconductor particles sensitized by a dye. In the photosensitive layer, the fine semiconductor particles act as a photosensitive substance to absorb light and conduct charge separation, thereby generating electrons and positive holes. In the dye-sensitized, fine semiconductor particles, the light absorption and the generation of electrons and positive holes are primarily caused by the dye, and the fine semiconductor particles receive and convey electrons or positive holes. The semiconductor used in the present invention is preferably an *n*-type semiconductor, in which conductor electrons act as carriers under a photo-excitation condition to generate anode current.

### (1) Semiconductor

Used as the semiconductor may be simple semiconductor such as silicon and germanium; a III-V series compound semiconductor; a metal chalcogenide such as a metal oxide, a metal sulfide, a metal selenide and a composite thereof; a compound having a perovskite structure such as strontium titanate, calcium titanate, sodium titanate, barium titanate and potassium niobate; etc.

Preferable examples of the metal chalcogenide include oxides of titanium, tin, zinc, iron, tungsten, zirconium, hafnium, strontium, indium, cerium, yttrium, lanthanum, vanadium, niobium and tantalum; sulfides of cadmium, zinc, lead, silver, antimony and bismuth; selenides of cadmium or lead; cadmium telluride; etc. Additionally, compound semiconductors such as phosphides of zinc, gallium, indium or cadmium, selenides of gallium-arsenic or copper-indium, copper-indium sulfide, etc. may be used in the present invention. Further, composite semiconductors such as MₓO_{y}S_{z} and M₁ₓM_{2y}O_{z} are also preferably used in the present invention, wherein M, M₁ and M₂ independently represent a metal atom, O represents an oxygen atom, S represents a sulfur atom, and x, y and z represent numbers combined with each other to form a neutral molecule.

Specific examples of the semiconductor are preferably Si, TiO₂, SnO₂, Fe₂O₃, WO₃, ZnO, Nb₂O₅, CdS, ZnS, PbS, Bi₂S₃, CdSe, CdTe, SrTiO₃, GaP, InP, GaAs, CuInS₂ and CuInSe₂, more preferably TiO₂, ZnO, SnO₂, Fe₂O₃, WO₃, Nb₂O₅, CdS, PbS, CdSe, SrTiO₃, InP, GaAs, CuInS₂ and CuInSe₂, further preferably TiO₂ and Nb₂O₅, and particularly TiO₂. TiO₂ preferably contains 70% by volume or more, particularly 100% by volume, of an anatase-type crystal structure. The semiconductor is preferably doped with a metal to increase electron conductivity thereof. This metal is preferably divalent or trivalent. Further, the semiconductor is preferably doped with a monovalent metal to prevent a reverse current from the semiconductor to the charge transfer layer.

The semiconductor used in the present invention may have a monocrystalline or polycrystalline structure. From the viewpoints of production cost, stable supply of raw materials, energy-payback time, etc., the semiconductor is preferably polycrystalline, particularly a porous layer of fine semiconductor particles. The photosensitive layer may partly contain an amorphous semiconductor.

The particle size of the fine semiconductor particles is generally on the level of nm to µm. The average size of primary semiconductor particles, which is determined by averaging the diameters of circles equivalent to projected areas thereof, is preferably 5 to 200 nm, more preferably 8 to 100 nm. Further, the average size of secondary semiconductor particles in dispersion is preferably 0.01 to 30 µm.

Two or more of the fine semiconductor particles having different particle size distributions may be used in combination for the photosensitive layer. In this case, the average particle size of the smaller particles is preferably 25 nm or less, more preferably 10 nm or less. To improve a light-capturing rate of the photoelectric conversion device by scattering ray of incident light, the fine semiconductor particles having a large particle size, e.g. approximately 100 to 300 nm in diameter, may be used for the photosensitive layer.

Two or more kinds of the fine semiconductor particles may be used in combination for the photosensitive layer. In this case, one is preferably TiO₂, ZnO, Nb₂O₅ or SrTiO₃ and the other is preferably SnO₂, Fe₂O₃ or WO₃. More preferred combinations are ZnO and SnO₂, ZnO and WO₃, ZnO and SnO₂ and WO₃, etc. When two or more fine semiconductor particles are combined, their particle sizes may differ. Particularly preferred is a combination of larger-diameter particles of TiO₂, ZnO, Nb₂O₅ or SrTiO₃ and smaller-diameter particles of SnO₂, Fe₂O₃ or WO₃. The larger-diameter particles are preferably 100 nm or more in an average size, while the smaller-diameter particles are preferably 15 nm or less in an average size.

Preferably usable to produce the fine semiconductor particles are sol-gel methods described in Sumio Sakka, "Science of Sol-Gel Method," issued by Agune Shofusha (1998), and "Thin Film-Coating Technology by Sol-Gel Method" (1995) issued by the Technical Information Association, etc.; and gel-sol methods described in Tadao Sugimoto, "Synthesis of Mono-Dispersion Particles and Control of Their Size and Form by Novel Gel-Sol Method," and MATERIA, Vol. 35, No. 9, pages 1012 to 1018 (1996). Also preferable is a method developed by Degussa, which comprises preparing oxides by subjecting chlorides to a high-temperature hydrolysis in an oxyhydrogen salt.

In the case of using the fine semiconductor particles of TiO₂, the above-described sol-gel methods, other sol-gel methods, and high-temperature hydrolysis methods in oxyhydrogen salts of chlorides are preferably used, and a sulfuric acid method and a chlorine method described in Manabu Seino, "Titanium oxide - Properties and Applied Technologies," issued by Gihodo Shuppan, (1997) may also be used. Further preferable as the sol-gel method are those described in Christophe J. Barb'e, et al, the Journal of American Ceramic Society, Vol. 80, No. 12, pages 3157 to 3171 (1997), and Burnside, et al, Chemistry of Materials, Vol. 10, No. 9, pages 2419 to 2425.

### (2) Layer of fine semiconductor particles

The fine semiconductor particles may be applied onto the conductive support by a method for coating a conductive support with a dispersion or a colloidal solution containing fine semiconductor particles, in addition to the above-mentioned sol-gel method. A wet-type film production method is relatively advantageous for the mass production of the photoelectric conversion device, the properties of a liquid containing fine semiconductor particles, the feasibility of conductive supports, etc. Typical examples of such a wet-type film production method are a coating method, a printing method, an electrolytic deposition method and an electrodeposition method. Further, the layer of fine semiconductor particles may be formed by a metal oxidation method, an LPD method for precipitating a metal from a metal solution by ligand exchange, a sputtering method, a vapor deposition method, a CVD method, or an SPD method for forming a metal oxide by thermally decomposing a metal oxide precursor sprayed onto a heated substrate.

The dispersion containing fine semiconductor particles may be prepared by crushing the semiconductor in a mortar, by dispersing the semiconductor while grinding in a mill, or by synthesizing and precipitating the fine semiconductor particles in a reaction solvent, etc. in addition to the above-mentioned sol-gel method.

Usable as dispersion solvents are water or organic solvents such as methanol, ethanol, isopropyl alcohol, citronellol, terpineol, dichloromethane, acetone, acetonitrile, ethyl acetate, etc. A Polymer such as polyethylene glycol, hydroxyethylcellulose and carboxymethylcellulose, a surfactant, an acid, a chelating agent, etc. may be used as a dispersing agent, if necessary. Polyethylene glycol is preferably added to the dispersion, because changing the molecular weight of the polyethylene glycol makes it possible to control the viscosity of the dispersion and the porosity of the resultant layer of fine semiconductor particles, and form a layer of fine semiconductor particles resistant to peeling.

Preferable coating methods include application methods such as a roller method and a dipping method, metering methods such as an air-knife method and a blade method, etc. Further, preferable as methods for applying and metering in the same portion are a wire-bar method disclosed in JP 58-4589 B, a slide-hopper method described in U. S. Patents 2,681,294, 2,761,419 and 2,761,791, an extrusion method, a curtain method, etc. Further preferable as commonly usable methods are a spin method and a spray method. Usable as wet printing methods are three major printing methods by relief printing, offset printing and gravure printing, as well as an intaglio printing method, a gum printing method, a screen printing method, etc. A film production method may be selected from these methods depending on the viscosity of the dispersion and the desired wet thickness.

The layer of fine semiconductor particles is not limited to a single layer. Dispersions comprising fine semiconductor particles having different particle sizes may be coated to form a multi-layer. Alternatively, dispersions containing different fine semiconductor particles, binders or additives may be coated to form a multi-layer. The multi-layer coating is effective, when it is impossible to form a layer having a sufficient thickness by one coating step.

Generally, the thicker the layer of fine semiconductor particles (having an equal thickness to that of the photosensitive layer), the higher the light-capturing rate, because a larger amount of dye is incorporated therein per a unit-projected area. In this case, however, there is large loss owing to recombination of electric charges because of increased diffusion distance of the generated electrons. Thus, the preferable thickness of the layer of fine semiconductor particles is 0.1 to 100 µm. In the photoelectric cell, the thickness of the layer of fine semiconductor particles is preferably 1 to 30 µm, more preferably 2 to 25 µm. The amount of the fine semiconductor particles applied to the substrate is preferably 0.5 to 100 g, more preferably 3 to 50 g, per 1 m² of the substrate.

After applying the fine semiconductor particles to the conductive support, the particles are preferably subjected to a heat treatment, thereby bringing them into electronic contact with each other, and increasing the strength of the resultant coating and their adhesion to the support. The heating temperature is preferably 40 to 700°C, more preferably 100 to 600°C. The heating time is preferably 10 minutes to 10 hours. High-temperature treatment is not preferable to a substrate having low melting point or softening point, such as a polymer film substrate, because it tends to deteriorate such a substrate. The heat treatment is preferably carried out at as low a temperature as possible, for example, 50 to 350°C, from the viewpoint of cost. Such a low-temperature heat treatment can be made possible by using a layer containing as fine semiconductor particles as 5 nm or less in average size, a mineral acid, a metal oxide precursor, etc. Further, the heat treatment may be carried out while applying an ultraviolet ray, an infrared ray, a microwave, an electric field, an ultrasonic wave, etc. to the fine semiconductor particles, to lower the heating temperature. To remove unnecessary organic compounds, etc., the heat treatment is preferably carried out in combination with evacuation, an oxygen plasma treatment, and washing with pure water, a solvent or a gas, etc.

After the heat treatment, the layer of fine semiconductor particles may be subjected to chemical metal plating using an aqueous titanium tetrachloride solution, etc. or electrochemical metal plating using an aqueous titanium trichloride solution, etc., to increase the surface area and purity of the fine semiconductor particles, thereby improving the efficiency of injecting electrons into the particles from the dye. Further, to prevent reverse current from the fine semiconductor particles to the charge transfer layer, it is effective for the fine semiconductor particles to adsorb other low-electron conductivity organic compounds than the dye. The organic compound adsorbed preferably has a hydrophobic group.

The layer of fine semiconductor particles preferably has a large surface area to adsorb a large amount of dye. The layer of fine semiconductor particles coated onto the substrate has a surface area of preferably 10 times or more, more preferably 100 times or more, as its projected area. The upper limit of the surface area is generally about 1000 times, though it is not particularly limited.

### (3) Dye

Though any compound capable of absorbing visible and near infrared rays to sensitize the semiconductor may be used as the dye for the photosensitive layer, preferable examples of such dyes include metal complex dyes, methine dyes, porphyrin-type dyes and phthalocyanine dyes. Two or more kinds of dyes may be used in combination to obtain a large photoelectric conversion wavelength region and high photoelectric conversion efficiency. In the case of using a plurality of dyes, the kinds and mixing ratios of the dyes may be determined depending on the wavelength region and strength distribution of the light source.

The dye preferably has an appropriate interlocking group for interacting with the surfaces of the fine semiconductor particles. Preferred interlocking groups include acidic groups such as -COOH, -OH, -SO₃H, -P(O)(OH)₂ and -OP(O)(OH)₂, and π-conductive chelating groups such as oxime, dioxime, hydroxyquinoline, salicylate and α-ketoenolate groups. Particularly preferable among them are -COOH, -P(O)(OH)₂ and -OP(O)(OH)₂. The interlocking group may form a salt with an alkali metal, etc. or an intramolecular salt.

The preferred dyes for the photosensitive layer are specifically described below.

### Metal complex dyes

When the dye is a metal complex dye, the dye is preferably a metallophthalocyanine dye, a metalloporphyrin dye or a ruthenium complex dye, particularly a ruthenium complex dye. The ruthenium complex dyes described in United States Patents 4,927,721, 4,684,537, 5,084,365, 5,350,644, 5,463,057 and 5,525,440, JP 7-249790 A, JP 10-504512 A and JP 2000-26487 A, WO 98/50393, etc. may be used in the present invention.

The ruthenium complex dye is preferably represented by the following formula (IV):

(A₁)ₚRu(B-a)(B-b)(B-c) (IV),

wherein A₁ represents a unidentate of SCN; *p* is an integer of 2; and B-a and B-b without B-c are the same organic ligands represented by the following formula B-1.

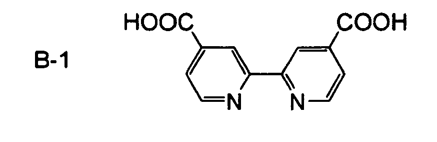

A preferred example of the metal complex dyes is illustrated below without intention of restriction.

(A₁)ₚRu(B-a)(B-b)(B-c) (IV),

| R | A₁ | p | B-a | B-b | B-c | R₆ |
|---|---|---|---|---|---|---|
| R-1 | SCN | 2 | B-1 | B-1 | - | - |

### (4) Adsorption of dye onto fine semiconductor particles

The dye may be adsorbed to the fine semiconductor particles by soaking the conductive support having the well-dried layer of fine semiconductor particles in a dye solution, or by coating the dye solution to the layer of fine semiconductor particles. In the former case, a soaking method, a dipping method, a roller-coating method, an air-knife method, etc. may be used. In the soaking method, the dye may be adsorbed at a room temperature or while refluxing as described in JP 7-249790 A. As a coating method of the latter, a wire-bar method, a slide-hopper method, an extrusion method, a curtain method, a spin-coating method, a spraying method, etc. may be used.

Preferable examples of solvents for dissolving the dye include alcohols such as methanol, ethanol, *t*-butanol and benzyl alcohol; nitriles such as acetonitrile, propionitrile and 3-methoxypropionitrile; nitromethane; halogenated hydrocarbons such as dichloromethane, dichloroethane, chloroform and chlorobenzene; ethers such as diethylether and tetrahydrofuran; dimethylsulfoxide; amides such as *N*,*N*-dimethylformamide and *N*,*N*-dimethylacetamide; *N*-methylpyrrolidone; 1,3-dimethylimidazolidinone; 3-methyloxazolidinone; esters such as ethyl acetate and butyl acetate; carbonates such as diethyl carbonate, ethylene carbonate and propylene carbonate; ketones such as acetone, 2-butanone and cyclohexanone; hydrocarbons such as hexane, petroleum ether, benzene and toluene; and mixtures thereof.

The total amount of the dye adsorbed is preferably 0.01 to 100 mmol per a unit surface area (1 m²) of the layer of fine semiconductor particles. The amount of the dye adsorbed onto the fine semiconductor particles is preferably 0.01 to 1 mmol per 1 g of the fine semiconductor particles. With this adsorption amount of the dye, the semiconductor can be sufficiently sensitized. Too small an amount of the dye results in insufficient sensitization. On the other hand, if the amount of the dye is excessive, the dye not adsorbed onto the fine semiconductor particles is free, thereby reducing the sensitization of the fine semiconductor particles. To increase the adsorption amount of the dye, it is preferable that the layer of fine semiconductor particles is subjected to a heat treatment before the dye is adsorbed thereonto. After the heat treatment, it is preferable for the layer of fine semiconductor particles to quickly adsorb the dye while it is still at 60 to 150°C without returning to room temperature, to prevent water from being adsorbed onto the layer of fine semiconductor particles.

To weaken interaction such as association between the dyes, a colorless compound may be co-adsorbed onto the fine semiconductor particles together with the dye. The colorless compound preferably has surface activity, and examples thereof include steroid compounds having a carboxyl group such as chenodeoxycholic acid and sulfonates shown below.

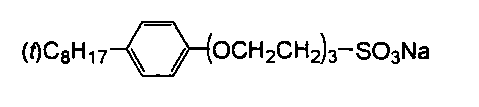

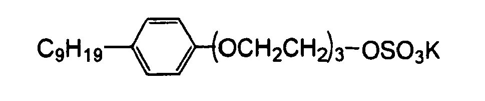

The dye not adsorbed on the layer of fine semiconductor particles is preferably removed by washing immediately after the dye adsorption process. The washing is preferably carried out by a wet-type washing bath with a polar solvent such as acetonitrile or an organic solvent such as alcohol. The surfaces of the fine semiconductor particles may be treated with an amine compound or a quaternary salt thereof after the dye adsorption process. The amine compound is preferably pyridine, 4-*t*-butylpyridine, polyvinylpyridine, etc., and the quaternary salt is preferably tetrabutylammonium iodide, tetrahexylammonium iodide, etc. The amine compound or the quaternary salt may be used alone if it is liquid, or may be used in the form of a solution in an organic solvent.

### (C) Charge transfer layer

The charge transfer layer comprises the electrolytic composition of the present invention described above. The charge transfer layer may be formed by any of the following two methods. One method is to attach a counter electrode to a photosensitive layer beforehand and cause a material for a charge transfer layer in a liquid state to penetrate into a gap therebetween. Another method is to directly form a charge transfer layer on a photosensitive layer, and then form a counter electrode thereon.

In the former method, the charge transfer material may be caused to penetrate into the gap by a normal pressure process utilizing capillarity, or by a reduced pressure process where the material is sucked into the gap to replace a gas phase therein with a liquid phase.

In the case of forming a wet charge transfer layer by the latter method, a counter electrode is attached while the charge transfer layer is still wet, to prevent the leakage of a liquid in edge portions. In the case of a gel electrolyte, it is applied in a wet state and solidified by polymerization. In this case, the counter electrode may be attached after drying and solidification. As a method for applying a wet organic hole-transporting material and a gel electrolyte apart from the electrolyte solution, the same methods as those described above for forming a layer of fine semiconductor particles and applying a dye may be used.

In the case of a solid electrolytic composition and a solid hole transporting material, the charge transfer layer may be formed by a dry film-forming method such as a vacuum deposition method and a CVD method, followed by attaching a counter electrode thereto. The organic hole-transporting material may be introduced into the photosensitive layer by a vacuum deposition method, a casting method, a coating method, a spin-coating method, a soaking method, an electrolytic polymerization method, a photo-polymerization method, etc. The inorganic hole-transporting material may be introduced into the photosensitive layer by a casting method, a coating method, a spin-coating method, a soaking method, an electrolytic deposition method, an electroless deposition method, etc.

The water content of the charge transfer layer is preferably 10,000 ppm or less, more preferably 2,000 ppm or less, and particularly 100 ppm or less.

### (D) Counter electrode

Like the above conductive support, the counter electrode may be an electrically conductive layer alone or a laminate of the electrically conductive layer and the substrate. Examples of electrically conductive materials used for the counter electrode include metals such as platinum, gold, silver, copper, aluminum, magnesium and indium; carbon; and electrically conductive metal oxides such as indium-tin composite oxides and fluorine-doped tin oxides. Preferable among them are platinum, gold, silver, copper, aluminum and magnesium. The substrate for the counter electrode is preferably a glass or plastic plate, to which the above electrically conductive material is applied by coating or vapor deposition. The counter electrode layer preferably has a thickness of 3 nm to 10 µm, although the thickness is not particularly limited. The surface resistance of the counter electrode layer is desirably as low as possible. The surface resistance is preferably 50 Ω/square or less, more preferably 20 Ω/square or less.

Because light may be irradiated from either one or both sides of the conductive support and the counter electrode, at least one of them should be substantially transparent to enable light to reach the photosensitive layer. To improve the efficiency of power generation, it is preferable that the conductive support is substantially transparent to permit light to pass therethrough. In this case, the counter electrode is preferably reflective to light. Such a counter electrode may be a glass or plastic plate vapor-deposited with a metal or an electrically conductive oxide, or a thin metal film.

The counter electrode may be formed by coating, plating or vapor-deposition (PVD, CVD, etc.) of an electrically conductive material directly onto the charge transfer layer, or by attaching the electrically conductive layer formed on the substrate to the charge transfer layer. Like in the conductive support, it is preferable to use a metal lead to reduce the resistance of the counter electrode, particularly when the counter electrode is transparent. Preferable materials and forming methods of the metal lead, decrease in incident light quantity, etc. are the same as those in the conductive support.

### (E) Other layers

A thin, dense semiconductor film is preferably preformed as an undercoating layer between the conductive support and the photosensitive layer, to prevent short-circuiting between the counter electrode and the conductive support. The prevention of short-circuiting by this undercoating layer is effective particularly in the case of the charge transfer layer comprising the electron-transporting material or the hole-transporting material. The undercoating layer is made of TiO₂, SnO₂, Fe₂O₃, WO₃, ZnO or Nb₂O₅, more preferably TiO₂. The undercoating layer may be formed by a spray-pyrolysis method described in Electrochim. Acta, 40, 643 to 652 (1995), a sputtering method, etc. The thickness of the undercoating layer is preferably 5 to 1000 nm, more preferably 10 to 500 nm.

Functional layers such as a protective layer and a reflection-preventing layer may be formed on either one or both of the conductive support and the counter electrode acting as electrodes, between the electrically conductive layer and the substrate, or in the substrate. The functional layers may be formed by a coating method, a vapor deposition method, an attaching method, etc. depending on their materials.

### (F) Interior structure of photoelectric conversion device

As described above, the photoelectric conversion device may have various interior structures depending on its use. It is classified into two major structures, one allowing light incidence from both faces, and another allowing it from only one side. Figs. 2 to 9 illustrate the interior structures of the photoelectric conversion device, to which the present invention is preferably applicable.

In the structure illustrated in Fig. 2, a photosensitive layer 20 and a charge transfer layer 30 are formed between an electrically conductive, transparent layer 10a and a transparent counter electrode layer 40a. This structure allows light incidence from both sides of the device.

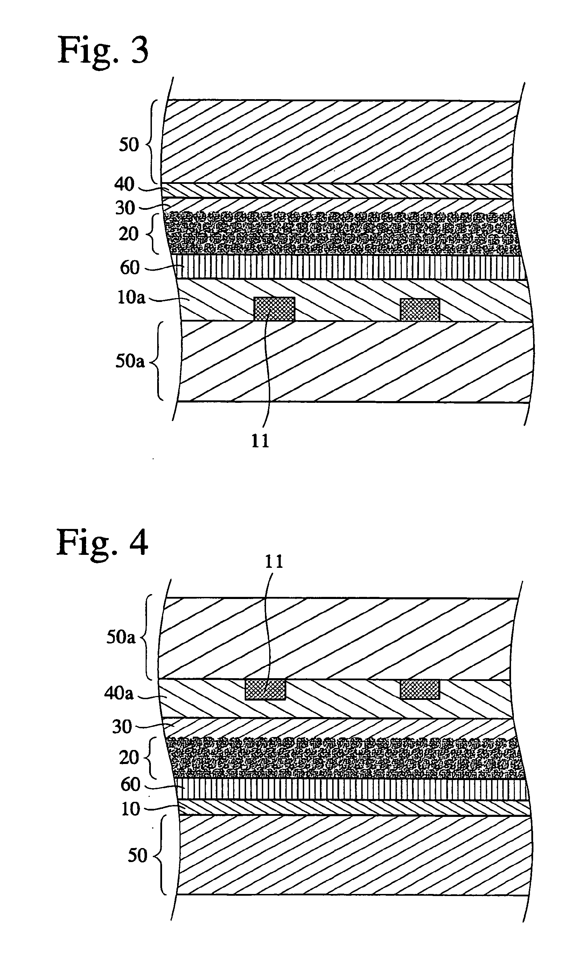

In the structure illustrated in Fig. 3, a transparent substrate 50a partially having a metal lead 11 is provided with an electrically conductive, transparent layer 10a, an undercoating layer 60, a photosensitive layer 20, a charge transfer layer 30 and a counter electrode layer 40 in this order, and further provided with a substrate 50 thereon. This structure allows light incidence from the side of the electrically conductive layer.

In the structure illustrated in Fig. 4, a substrate 50 having an electrically conductive layer 10 is provided with a photosensitive layer 20 via an undercoating layer 60, and then provided with a charge transfer layer 30 and a transparent counter electrode layer 40a thereon, and further provided with a transparent substrate 50a locally having a metal lead 11 inside. This structure allows light incidence from the side of the counter electrode.

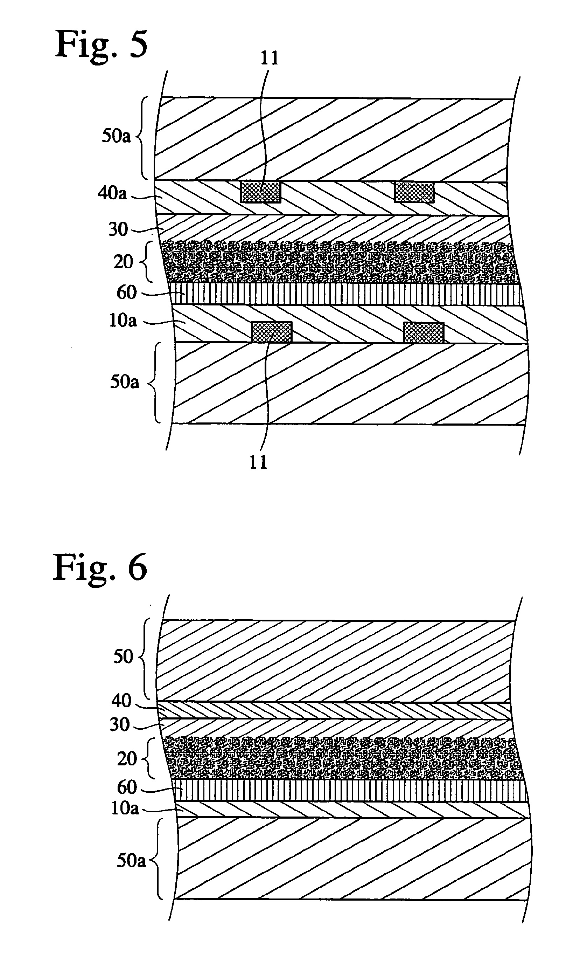

In the structure illustrated in Fig. 5, two transparent substrates 50a each partially having a metal lead 11 are provided with an electrically conductive transparent layer 10a or 40a, and then provided with an undercoating layer 60, a photosensitive layer 20 and a charge transfer layer 30 between the conductive layers. This structure allows light incidence from both sides of the photoelectric conversion device.

In the structure illustrated in Fig. 6, a transparent substrate 50a is provided with an electrically conductive transparent layer 10a, an undercoating layer 60, a photosensitive layer 20, a charge transfer layer 30 and a counter electrode layer 40 in this order, and then attached to a substrate 50. This structure allows light incidence from the side of the electrically conductive layer.

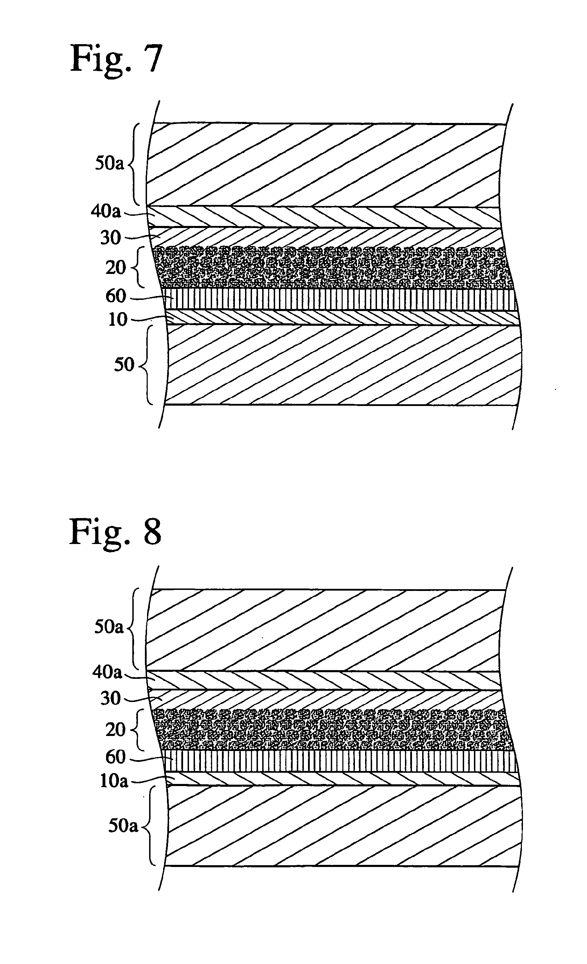

In the structure illustrated in Fig. 7, a substrate 50 is provided with an electrically conductive layer 10, an undercoating layer 60, a photosensitive layer 20, a charge transfer layer 30 and a transparent counter electrode layer 40a in this order, and then attached to a transparent substrate 50a. This structure allows light incidence from the side of the counter electrode.

In the structure illustrated in Fig. 8, a transparent substrate 50a is provided with an electrically conductive transparent layer 10a, an undercoating layer 60, a photosensitive layer 20, a charge transfer layer 30 and a transparent counter electrode layer 40a in this order, and then attached to a transparent substrate 50a. This structure allows light incidence from both sides of the photoelectric conversion device.

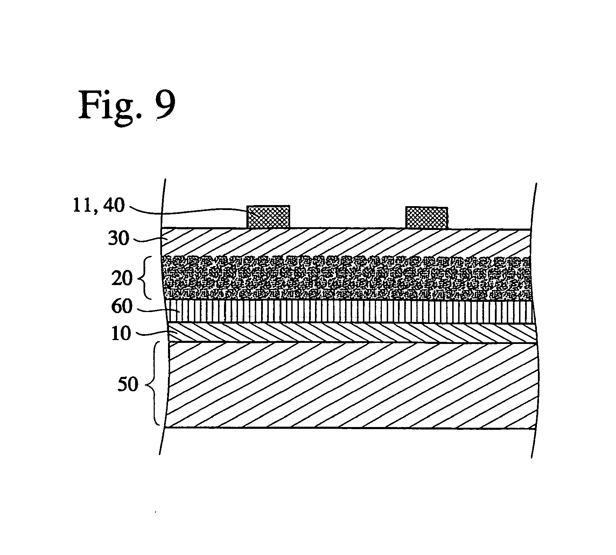

In the structure illustrated in Fig. 9, a substrate 50 is provided with an electrically conductive layer 10, an undercoating layer 60, a photosensitive layer 20, a solid charge transfer layer 30 in this order, and then partially provided with a counter electrode layer 40 or a metal lead 11. This structure allows light incidence from the side of the counter electrode.

### [3] Photoelectric cell and solar cell

The photoelectric cell of the present invention comprises the above photoelectric conversion device of the present invention to do job in an external circuit. Such a photoelectric cell that has the charge transfer layer comprising the ion-conductive electrolytic composition is generally referred to as a photo-electrochemical cell. The photoelectric cell intended for generating power with solar light is referred to as a solar cell.

The edges of the photoelectric cell are preferably sealed with a polymer or an adhesive, etc. to prevent the cell content from deteriorating and evaporating. A known external circuit may be connected to the conductive support and the counter electrode via a lead.

When the photoelectric conversion device of the present invention is used for a solar cell, the interior structure of the solar cell may be essentially the same as that of the photoelectric conversion device mentioned above. The dye-sensitized solar cell comprising the photoelectric conversion device of the present invention may substantially have the same module structure as a conventional solar cell module. In a general module structure of the solar cell, a cell is disposed on a substrate of metal, ceramic, etc. and covered with a packing resin, a protective glass, etc., whereby light is introduced from the opposite side of the substrate. The solar cell module may have a structure where the cell is placed on a substrate of a transparent material such as a tempered glass to introduce light from the transparent substrate side. Specifically, a superstraight-type module structure, a substrate-type module structure, a potting-type module structure, substrate-integrated module structure that is generally used in amorphous silicon solar cells, etc. are known as the solar cell module structure. The dye-sensitive solar cell of the present invention may have a module structure properly selected from the above structures depending on ends, locations and environment where it is used, and preferably has a module structure disclosed in JP 2000-268892 A.

The present invention will be explained in more detail with reference to Examples below without intention of restricting the scope of the present invention.

### Examples 1-11 and Reference Example 1-10, Comparative Examples 1-5

### 1. Synthesis of electrolyte salts

The electrolyte salts used in the electrolytic compositions were synthesized as follows. The structure of each electrolyte salt was identified by NMR and Mass spectrum.

### (1) Synthesis of D-18 for reference example 8

2.1 g of 3-(trimethylsilyl)-1-propane sulfonate chloride and 1.4 g of trifluoromethane sulfonamide were dissolved in 20 ml of dichloromethane, and 2.8 ml of triethylamine was added thereto. The resultant solution was then stirred for one hour at room temperature. After adding water to the resultant solution, an organic phase extracted by dichloromethane was concentrated under a reduced pressure to obtain an oily substance. 10 ml of an aqueous solution containing 0.2 g of lithium hydroxide was added to this oily substance, and the solvent was removed under a reduced pressure. The resultant residue was purified by a silica gel column (eluent: methanol/dichloromethane) to obtain 3.0 g of an electrolyte salt D-18. The mass spectrum of the electrolyte salt D-18 revealed that it had a mass (POSI) of 334.

### (2) Synthesis of D-1 for example 1

2.8 g of an electrolyte salt D-18 synthesized as in the above (1) and 1.6 g of 1-methyl-3-ethylimidazolium bromide were dissolved in 20 ml of acetonitrile and refluxed while heating for 30 minutes. After adding water to the resultant solution, an organic phase extracted by dichloromethane was concentrated under a reduced pressure to obtain an oily substance. This oily substance was purified by a silica gel column (eluent: methanol/dichloromethane) to obtain 1.6 g of an electrolyte salt D-1. The mass spectrum of the resultant electrolyte salt D-1 revealed that it had a mass (POSI) of 438.

### (3) Synthesis of D-11 for reference example 1

2.8 g of an electrolyte salt D-18 synthesized as in the above (1) and 1.6 g of 1-ethylpyridinium bromide were dissolved in 20 ml of acetonitrile and refluxed while heating for 30 minutes. After adding water to the resultant solution, an organic phase extracted by dichloromethane was concentrated under a reduced pressure to obtain an oily substance. This oily substance was purified by a silica gel column (eluent: methanol/dichloromethane) to obtain 1.5 g of an electrolyte salt D-11. The mass spectra of the resultant electrolyte salt D-11 revealed that it had a mass (POSI) of 435.

### 2. Preparation of titanium dioxide dispersion

Charged into a 200-ml stainless steel vessel lined with Teflon were 15 g of fine titanium dioxide particles ("Degussa P-25" available from Nippon Aerosil K. K.), 45 g of water, 1 g of a dispersant ("Triton X-100" available from Aldrich), and 30 g of zirconia beads (diameter: 0.5 mm, available from Nikkato K. K.). They were subjected to a dispersion treatment at 1,500 rpm for 2 hours by a sand-grinder mill available from Imex K. K. The zirconia beads were removed by filtration to obtain a titanium dioxide dispersion. The average particle diameter of the titanium dioxide particles in the dispersion was 2.5 µm. The particle diameter was measured by Master Sizer available from MALVERN.

### 3. Preparation of dye-sensitized TiO₂ electrode

The above titanium dioxide dispersion was applied to an electrically conductive glass having a fluorine-doped tin oxide layer (20 mm x 20 mm, "TCO Glass-U" available from Asahi Glass K. K. with a surface resistance of approximately 30 Ω/square) on its conductive surface by a glass bar. The amount of the fine semiconductor particles coated was 20 g/m². With an adhesive tape attached to part of the conductive surface (3 mm from the edge) of each conductive glass as a spacer, eight conductive glasses were arranged such that the adhesive tapes were positioned at both ends, to coat them with the titanium dioxide dispersion simultaneously. After peeling the adhesive tapes, the coated conductive glasses were air-dried at room temperature for a day. Each coated conductive glass was then placed in an electric furnace (muffle furnace "FP-32" available from Yamato Science K. K.), and calcined at 450°C for 30 minutes to obtain a TiO₂ electrode. After the TiO₂ electrode was taken out of the furnace and cooled, the electrode was immersed in a solution of Dye R-1 in ethanol (3 x 10⁻⁴ mol/l) for 3 hours and then in 4-*t*-butylpyridine for 15 minutes. It was then washed with acetonitrile and spontaneously dried to obtain a dye-sensitized TiO₂ electrode substrate.

### 4. Production of photoelectric conversion device

The above dye-sensitized TiO₂ electrode substrate (20 mm x 20 mm) was laminated on a platinum-vapor-deposited glass having the same size. Next, an electrolytic composition comprising 98% by mass of the following electrolyte salt Y-1 and 2% by mass of iodine was permeated into the TiO₂ electrode in a gap between the two glasses by capillarity, and sealed with an epoxy-based sealant to obtain a photoelectric conversion device of Comparative Example 1.

In the same manner as in Comparative Example 1 except for changing the electrolytic composition as shown in Table 1, the photoelectric conversion devices of Examples 1-11 and Reference Examples 1-10 and Comparative Examples 2 to 5 were produced. When the electrolytic composition had such high viscosity that they could not easily be permeated into a gap between the two glasses by capillarity, the electrolytic compositions were heated to 50°C and applied to the TiO₂ electrodes, which were then placed under reduced pressure, so that the electrolytic compositions were fully permeated into the TiO₂ electrodes with air therein removed. Each of them was then laminated with a platinum-vapor-deposited glass (counter electrode). The structures of the electrolyte salts Y-1 to Y-4 used in Examples, Reference Examples and Comparative Examples are illustrated below.

**Table 1**

| Photoelectric Conversion Device | Electrolytic Composition Containing 2% by Mass of Iodine |
|---|---|
| | Electrolyte Salt (% by mass) |
| Comp. Ex. 1 | Y-1 (98) |
| Comp. Ex. 2 | Y-1 (20) / Y-2 (78) |
| Comp. Ex. 3 | Y-1 (20) / Y-3 (78) |
| Comp. Ex. 4 | Y-1 (10) / Y-4 (88) |
| Comp. Ex. 5 | Y-1 (20) / Y-4 (78) |
| Example 1 | Y-1 (20) / D-1 (78) |
| Example 2 | Y-1 (20) / D-2 (78) |
| Example 3 | Y-1 (20) / D-3 (78) |
| Example 4 | Y-1 (20) / D-4 (78) |
| Example 5 | Y-1 (20) / D-5 (78) |
| Example 6 | Y-1 (20) / D-6 (78) |
| Example 7 | Y-1 (20) / D-7 (78) |
| Example 8 | Y-1 (20) / D-8 (78) |
| Example 9 | Y-1 (20) / D-9 (78) |
| Example 10 | Y-1 (20) / D-10 (78) |
| Ref. Ex. 1 | Y-1 (20) / D-11 (78) |
| Ref. Ex. 2 | Y-1 (20) / D-12 (78) |
| Ref. Ex. 3 | Y-1 (20) / D-13 (78) |
| Ref. Ex. 4 | Y-1 (20) / D-14 (78) |
| Ref. Ex. 5 | Y-1 (20) / D-15 (78) |
| Ref. Ex. 6 | Y-1 (20) / D-16 (78) |
| Ref. Ex. 7 | Y-2 (20) / D-17 (78) |
| Ref. Ex. 8 | Y-1 (20) / D-18 (78) |
| Ref. Ex. 9 | Y-1 (20) / D-19 (78) |
| Ref. Ex. 10 | Y-1 (20) / D-20 (78) |
| Example 11 | Y-1 (10) / D-1 (88) |

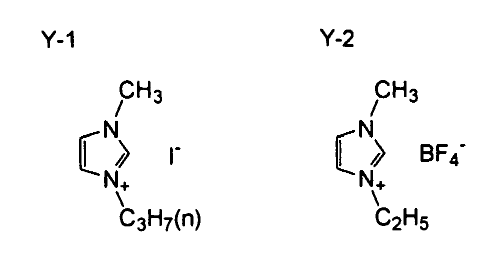

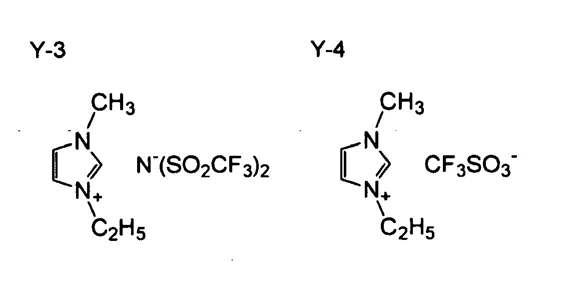

### 5. Measurement of photoelectric conversion efficiency

Each of the above photoelectric conversion devices of Examples 1-11 and Reference Examples 1-10 and Comparative Examples 1 to 5 was measured with respect to photoelectric conversion efficiency by an apparatus for measuring IPCE (incident photon to current conversion efficiency) available from Optel Co. The photoelectric conversion efficiency (IPCE) of each photoelectric conversion device at a wavelength at which it exhibited the maximum conversion ability is shown in Table 2 below.

**Table 2**

| Photoelectric Conversion Device | Photoelectric Conversion Efficiency (IPCE) (%) |
|---|---|
| Comp. Ex. 1 | 55 |
| Comp. Ex. 2 | 57 |
| Comp. Ex. 3 | 58 |
| Comp. Ex. 4 | 60 |
| Comp. Ex. 5 | 62 |
| Example 1 | 74 |
| Example 2 | 72 |
| Example 3 | 71 |
| Example 4 | 69 |
| Example 5 | 72 |
| Example 6 | 69 |
| Example 7 | 70 |
| Example 8 | 71 |
| Example 9 | 68 |
| Example 10 | 70 |
| Ref. Ex. 1 | 68 |
| Ref. Ex. 2 | 67 |
| Ref. Ex. 3 | 69 |
| Ref. Ex. 4 | 67 |
| Ref. Ex. 5 | 65 |
| Ref. Ex. 6 | 67 |
| Ref. Ex. 7 | 69 |
| Ref. Ex. 8 | 64 |
| Ref. Ex. 9 | 68 |
| Ref. Ex. 10 | 63 |
| Example 11 | 69 |

As shown in Table 2, the photoelectric conversion devices of Examples 1 to 11 each comprising the electrolyte salt (I) were superior in photoelectric conversion efficiency to those of Comparative Examples 1 to 5 each comprising a conventional electrolyte salt.

As explained above in detail, the electrolytic composition suitable for a dye-sensitized solar cell of the present invention is excellent in charge-transporting capability. The photoelectric conversion device comprising this electrolytic composition exhibits excellent photoelectric conversion properties. The photoelectric cell comprising such photoelectric conversion device is remarkably useful as a solar cell.

## Claims

1. A dye-sensitized solar cell comprising a photoelectric conversion device comprising a conductive support, a photosensitive layer (20), a charge transfer layer (30) and a counter electrode (40),
wherein
said conductive support comprises a conductive glass,
said photosensitive layer (20) comprises fine semiconductor particles (21) of TiO₂ sensitized by a dye (22), said counter electrode (40) is same as the conductive glass as those in the conductive support, **characterised in that**
said charge transfer layer (30) comprises an electrolytic composition comprising an electrolyte salt represented by the following general formula (I):
(Q₁)ₙ₁·(X)ₙ₂ (I),
wherein Q₁ represents an unsubstituted imidazolium cation, X represents an anion having a silicon atom, and each of n₁ and n₂ represents an integer of 1, wherein said X is represented by the following general formula (II): 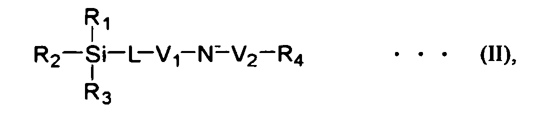 wherein each of R₁ to R₄ independently represents a substituent of a substituted or unsubstituted alkyl group having 1 to 2 carbon atoms, each of L, V₁ and V₂ independently represents a bivalent linking group, at least one of V₁ and V₂ represents -CO-, -SO-, -SO₂- or -PO(OR₅)-, and R₅ represents methyl group.

2. The dye-sensitized solar cell according to claim 1, wherein said conductive glass is a platinum-vapor-deposited glass.

3. The dye-sensitized solar cell according to claim 1, wherein said dye (22) is a ruthenium complex dye represented by the following formula (IV):
(A₁)ₚRu(B-a)(B-b)(B-c) (IV),
wherein A₁ represents SCN; *p* is an integer of 2; and B-a and B-b without B-c are the same organic ligands represented by the following formulae B-1.

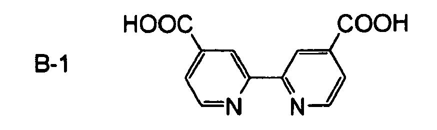

4. A compound represented by the following general formula (III): 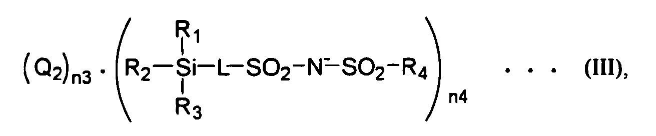 wherein Q₂ represents an unsubstituted imidazolium cation, each of R₁ to R₄ independently represents a substituent of a substituted or unsubstituted alkyl group having 1 to 2 carbon atoms, L represents a bivalent linking group, and each of n₃ and n₄ represents an integer of 1.

## Patentansprüche

1. Farbstoff-sensibilisierte Solarzelle, umfassend eine photoelektrische Umwandlungsvorrichtung, umfassend einen leitfähigen Träger, eine lichtempfindliche Schicht (20), eine Ladungsübertragungsschicht (30) und eine Gegenelektrode (40), wobei
der leitfähige Träger ein leitfähiges Glas umfasst,
die lichtempfindliche Schicht (20) feine Halbleiterteilchen (21) aus mit einem Farbstoff (22) sensibilisiertem TiO₂ umfasst,
die Gegenelektrode (40) die gleiche ist, wie das leitfähige Glas im leitfähigen Träger,
**dadurch gekennzeichnet, dass**
die Ladungsübertragungsschicht (30) eine Elektrolytzusammensetzung umfasst, umfassend ein Elektrolytsalz der allgemeinen Formel (I):
(Q₁)ₙ₁(X)ₙ₂ (I),
wobei Q₁ ein unsubstituiertes Imidazolium Kation bedeutet, X ein Anion mit einem Siliziumatom bedeutet, n₁ und n₂ jeweils eine ganze Zahl von 1 bedeuten, wobei X repräsentiert wird durch die nachstehende allgemeine Formel II: 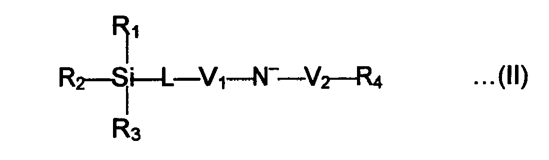 wobei jedes R₁ bis R₄ unabhängig einen Substituenten in Form einer substituierten oder unsubstituierten Alkylgruppe mit 1 bis 2 Kohlenstoffatomen bedeutet, jedes L, V₁ und V₂ unabhängig einer zweiwertige Verknüpfungsgruppe bedeutet, wenigstens eines von V₁ und V₂ für -CO-, -SO-, -SO₂- oder -PO(OR₅)- steht und R₅ eine Methylgruppe bedeutet.

2. Farbstoff-sensibilisierte Solarzelle nach Anspruch 1, wobei das leitfähige Glas ein mit Platin bedampftes Glas ist.

3. Farbstoff-sensibilisierte Solarzelle nach Anspruch 1, wobei der Farbstoff (22) ein Ruthenium-Komplexfarbstoff der nachstehenden Formel (IV) ist:
(A₁)ₚRu(B-a)(B-b)(B-c (IV),
wobei A₁ für SCN steht; p eine ganze Zahl von 2 ist; und B-a und B-b ohne B-c die gleichen organischen Liganden sind, repräsentiert durch die nachstehende Formel B-1.

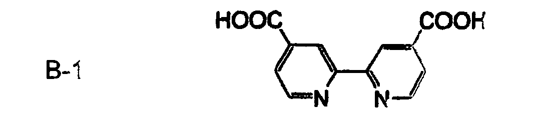

4. Verbindung gemäß der nachstehenden allgemeinen Formel (III): 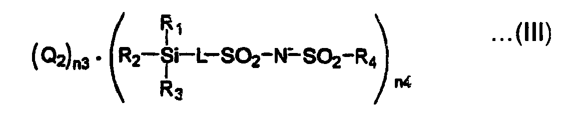 wobei Q₂ einen unsubstituiertes Imidazolium Kation bedeutet, jedes R₁ bis R₄ unabhängig einen Substituenten in Form einer substituierten oder unsubstituierten Alkylgruppe mit 1 bis 2 Kohlenstoffatomen bedeutet, L eine zweiwertige Verknüpfungsgruppe bedeutet, und jedes von n₃ und n₄ eine ganze Zahl von 1 ist.

## Revendications

1. Cellule solaire sensibilisée par un colorant comprenant un dispositif de conversion photoélectrique comprenant un support conducteur, une couche photosensible (20), une couche de transfert de charge (30) et une contre-électrode (40),
dans laquelle
ledit support conducteur comprend un verre conducteur,
ladite couche photosensible (20) comprend de fines particules semi-conductrices (21) de TiO₂ sensibilisées par un colorant (22), ladite contre-électrode (40) est identique au verre conducteur utilisé dans le support conducteur,
**caractérisée en ce que**
ladite couche de transfert de charge (30) comprend une composition électrolytique comprenant un sel d'électrolyte représenté par la formule générale (I) suivante :
(Q₁)ₙ₁·(X)ₙ₂ (I),
dans laquelle Q₁ représente un cation imidazolium non substitué; X représente un anion ayant un atome de silicium, et chacun des n₁ et n₂ représente un nombre entier valant 1, ledit X étant représenté par la formule générale (II) suivante : 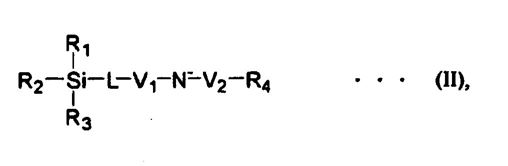 dans laquelle chacun des R₁ à R₄ représente indépendamment un substituant d'un groupe alkyle substitué ou non ayant 1 à 2 atomes de carbone, chacun des L, V₁ et V₂ représente indépendamment un groupe de liaison bivalent, au moins un des V₁ et V₂ représentant -CO-, -SO-, -SO₂- ou -PO(OR₅)-, où R₅ représente un groupe méthyle.

2. Cellule solaire sensibilisée par un colorant selon la revendication 1, dans laquelle ledit verre conducteur est un verre obtenu par déposition en phase vapeur de platine.

3. Cellule solaire sensibilisée par un colorant selon la revendication 1, dans laquelle ledit colorant (22) est un colorant complexe de ruthénium représenté par la formule (IV) suivante :
(A₁)ₚRu(B-a)(B-b)(B-c) (IV),
dans laquelle A₁ représente SCN ; *p* est un nombre entier valant 2 ; et B-a et B-b sans B-c sont des ligands organiques identiques représentés par la formule B-1 suivante :

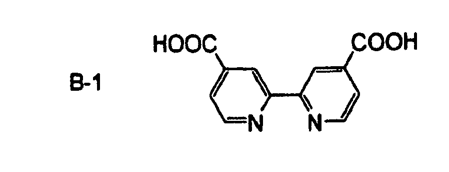

4. Composé représenté par la formule générale (III) suivante : 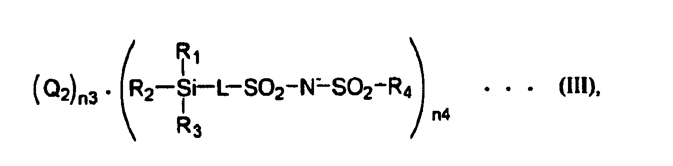 dans laquelle Q₂ représente un cation imidazolium non substitué, chacun des R₁ à R₄ représente indépendamment un substituant d'un groupe alkyle substitué ou non ayant 1 à 2 atomes de carbone, L représente un groupe de liaison bivalent, et chacun des n₃ et n₄ représente un nombre entier valant 1.
